# EUROPEAN PATENT APPLICATION

(11) **EP 3 885 428 A1**
(43) Date of publication of application: **29.09.2021**
(21) Application number: 19905997.3
(22) Date of filing: 25.12.2019
(51) Int. Cl.: C12M 1/00, C12M 1/10, C12M 3/00

(54) **PERFUSION CULTURE APPARATUS AND CENTRIFUGAL SEPARATOR**

(30) Priority: 27.12.2018 JP 2018248966
(71) Applicant: Able Corporation, Tokyo 162-0812 (JP)
(72) Inventor: ISHIKAWA Yoichi, Tokyo 162-0812 (JP)
(74) Representative: reuteler & cie SA
(86) International application number: PCT/JP2019/051646
(87) International publication number: WO 2020/138506

(57) **Abstract**

Provided are a perfusion culture apparatus which can be utilized in long-duration culturing and which has a simple mechanism and is easy to use; and a centrifugal separator. The perfusion culture apparatus has a centrifugal separation tank 30 for centrifugally separating a culture solution W delivered from a culture tank 10, and a discharge mechanism 60 for causing a centrifugally separated supernatant of the culture solution to be discharged from the centrifugal separation tank. The centrifugal separation tank is provided with a cylindrical inner wall surface 32, and is configured so as to rotate about the center axis of the inner wall surface and thereby centrifugally separate the culture solution. The discharge mechanism includes a suction pipe 62 provided with a first end portion 64 which faces the inner wall surface and is disposed closer to the center axis than the culture solution during centrifugal separation, and a second end portion 66 which is disposed outside the centrifugal separation tank. In a state in which the centrifugal separation tank is rotated and the culture solution is centrifugally separated, the air in the centrifugal separation tank is sucked from the first end portion of the suction pipe, and the centrifugally separated supernatant of the culture solution is thereby discharged, together with the air, from the second end portion.

## Description

### [Technical Field]

This invention relates to a perfusion culture apparatus, particularly, a perfusion culture apparatus utilizing centrifugation, and a centrifugal separator.

### [Background Art]

In the field of drug discovery and food products, cell culture techniques are used to produce a target product, and if the cell density in a culture solution can be increased, production efficiency of the target product can be enhanced.

In the cell culture process, cells produce various metabolites, causing inhibition of cell growth or substance production, which may reduce cell culture efficiency.

### [Prior Art Publications]

### [Patent Documents]

Patent Document 1: Japanese Patent Application Publication S52-114085
Patent Document 2: Japanese Patent Application Publication S63-252558

### [Summary of the Invention]

### [Problem to be Solved by the Invention]

In the case of utilizing a filtration membrane for cell separation, there is a possibility that the filtration membrane may be clogged, which is necessary to devise measures for its prevention for using the filtration membrane for long-term culture. Further, it was difficult to simplify the apparatus when centrifugal force is used to separate cells.

It is an object of this invention to provide a perfusion culture apparatus which can be used for long-term culture and has a simple mechanism and which is easy to handle, and a centrifugal separator.

### [Means for Solving the Problem]

A perfusion culture apparatus according to this invention includes a centrifugal separation tank for centrifuging a culture solution delivered from a culture tank, and a discharge mechanism for discharging a centrifugation supernatant of the culture solution from the centrifugal separation tank, in which the centrifugal separation tank has a cylindrical inner wall surface and is configured to rotate about a center axis of the inner wall surface to centrifuge the culture solution, in which the discharge mechanism includes a suction pipe having a first end portion arranged closer to the center axis than the culture solution during a centrifugation process and opposing the inner wall surface, and a second end portion arranged outside the centrifugal separation tank, in which, with the centrifugal separation tank being rotated and the culture solution being centrifuged, the air in the centrifugal separation tank is suctioned from the first end portion of the suction pipe, so that the centrifugation supernatant of the culture solution is discharged from the second end portion together with the air.

In the perfusion culture apparatus, the culture solution may be centrifuged while delivering the culture solution to the centrifugal separation tank, or the culture solution may be centrifuged while stopping delivery of the culture solution to the centrifugal separation tank. The perfusion culture apparatus may further include a housing configured to allow introduction of sterile air for housing the centrifugal separation tank, and the suction pipe may be configured to allow adjustment of a distance between the first end portion and the inner wall surface. Further, the suction pipe may be configured to allow the first end portion to move toward the inner wall surface while suctioning the air, or may further include a recovery mechanism for recovering a residue of the culture solution from the rotation tank.

The centrifugal separator according to this invention includes a centrifugal separation tank for centrifuging a processing solution, and a discharge mechanism for discharging a centrifugation supernatant of the processing solution from the centrifugal separation tank, in which the centrifugal separation tank has a cylindrical inner wall surface and is configured to rotate about a center axis of the inner wall surface to centrifuge the processing solution, in which the discharge mechanism includes a suction pipe having a first end portion arranged closer to the center axis than the processing solution during centrifugation and opposed to the inner wall surface, and a second end portion arranged outside the centrifugal separation tank, in which, with the centrifugal separation tank being rotated and the processing solution being centrifuged, the air in the centrifugal separation tank is suctioned from the first end portion of the suction pipe, so that the centrifugation supernatant of the processing solution is discharged from the second end portion together with the air.

### [Effect of the Invention]

According to this invention, it is possible to provide a perfusion culture apparatus and a centrifugal separator capable of easily discharging a centrifugation supernatant, and having a simple mechanism and which is easy to handle.

### [Brief Description of the Drawings]

[FIG. 1] FIG. 1 is a diagram for explaining the perfusion culture apparatus according to this embodiment.
[FIG. 2] FIG. 2 is a diagram for explaining the irrigation culture step according to this embodiment.
[FIG. 3] FIG. 3 is a diagram for explaining the perfusion culture apparatus according to a modified example.
[FIG. 4] FIG. 4 is a diagram for explaining the perfusion culture apparatus according to a modified example.

### [Embodiments for Carrying Out the Invention]

Embodiments to which this invention is applied will be described below. However, this invention is not limited to the following embodiments. Namely, not all configurations described in the following embodiments are essential to this invention. This invention also includes any combination of those given below.

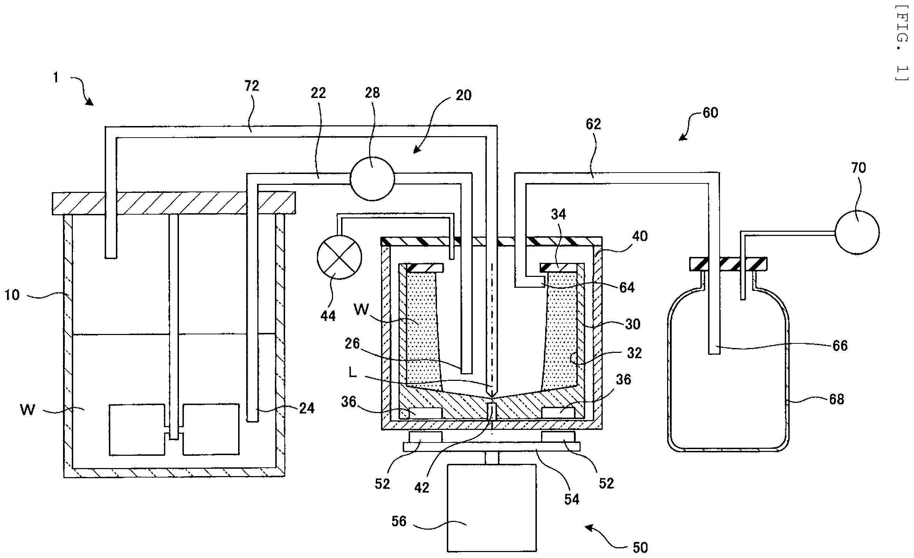

First, the configuration of a perfusion culture apparatus 1 according to an embodiment of this invention will be described with reference to FIG. 1. The perfusion culture apparatus 1 is configured as an apparatus for culturing various cells (e.g., cells of animals, insects, plants, etc., microorganisms, bacteria, etc.) in a culture solution.

The perfusion culture apparatus 1 according to this embodiment includes a culture tank 10. The culture tank 10 is a member for holding a culture solution W in which cells to be cultured are dispersed, and has various mechanisms for maintaining the culture solution W in a desired state. By adjusting the state of the culture solution W in the culture tank 10, the cells can be appropriately cultured in the culture solution W so that the target object can be produced.

Although not specifically shown, the culture tank 10 can be configured to include various mechanisms (ancillary equipment) for appropriately controlling the culture process. For example, the apparatus may be configured to include an equipment (ancillary equipment) such as a measuring device for measuring the state (dissolved oxygen, pH, temperature, etc.) of the culture solution W, a measuring device for measuring the degree of progress of the culture (device for measuring cell density, glucose concentration, etc.), an adjustment mechanism for adjusting the state of the culture solution W, a supply mechanism for supplying the culture solution W (fresh liquid medium) and nutrients, and a sampling mechanism for extracting the culture solution W.

The perfusion culture apparatus 1 according to this embodiment includes a liquid delivery unit 20. The liquid delivery unit 20 serves to deliver the culture solution W in the culture tank 10 to the centrifugal separation tank 30.

The liquid delivery unit 20 has a liquid delivery pipe 22. The liquid delivery pipe 22 has a first end portion 24 arranged in the culture tank 10 and a second end portion 26 arranged in the centrifugal separation tank 30. The liquid delivery unit 20 also has a pump 28. The culture solution W in the culture tank 10 can be delivered to the centrifugal separation tank 30 through a liquid delivery pipe 22 by a pump 28. Although the pump applicable to this embodiment is not particularly limited, it is preferable to select a pump that does not damage the cells. Alternatively, in place of the pump 28, an internal pressure of the culture tank 10 can be increased to deliver the culture solution W from the culture tank 10 to the centrifugal separation tank 30.

The perfusion culture apparatus 1 according to this embodiment includes a centrifugal separation tank 30. The centrifugal separation tank 30 serves to centrifuge the culture solution W delivered from the culture tank 10 for separation into a centrifugation supernatant and a precipitate. The centrifugal separation tank 30 has a cylindrical inner wall surface 32 and is configured to be rotatable about a center axis L of the inner wall surface 32. By rotation of the centrifugal separation tank 30 about the center axis L of the inner wall surface 32, the culture solution W stored in the centrifugal separation tank 30 is pressed against the inner wall surface 32 and centrifuged while being cylindrical.

The centrifugal separation tank 30 has a collar portion 34. The collar portion 34 is a disk-shaped member having an open center and is arranged at an upper end of the centrifugal separation tank 30. The collar portion 34 can prevent the culture solution W pressed against the inner wall surface 32 from spilling out of the centrifugal separation tank 30. The centrifugal separation tank 30 can be configured to have a baffle on its inner wall surface 32 (not shown). Since the movement of the culture solution W in the circumferential direction is restricted by the baffle, the centrifugal process can be efficiently performed.

In this embodiment, the centrifugal separation tank 30 is disposed within the housing 40. Concretely, the centrifugal separation tank 30 is held in a rotatable manner within the housing 40. For example, by inserting a shaft body 42 provided on a bottom surface of the housing 40 into a recess provided in a bottom surface of the centrifugal separation tank 30, and through attachment via a bearing (not shown), the centrifugal separation tank 30 can be made rotatable.

The housing 40 is configured to allow introduction of sterile air. For example, the housing 40 can be configured to introduce sterile air into its interior via a filter 44. In this embodiment, the filter 44 may be attached to the housing 40 via a check valve. Thus, an internal pressure of the housing 40 is increased, and the culture solution W held in the centrifugal separation tank 30 (residue of centrifugation) can be returned to the culture tank 10.

The perfusion culture apparatus 1 according to this embodiment includes a drive mechanism 50. The drive mechanism 50 serves to rotationally drive the centrifugal separation tank 30. The drive mechanism 50 has a magnet 52 and a holding body 54 for holding the magnet 52. The magnet 52 is opposed to a magnet 36 provided at the bottom of the centrifugal separation tank 30, and the holding body 54 is rotated by a motor 56 so that the centrifugal separation tank 30 can be rotated with the magnet 52 (holding body 54) .

The perfusion culture apparatus 1 according to this embodiment includes a discharge mechanism 60. The discharge mechanism 60 serves to discharge the culture solution W (centrifugation supernatant of culture solution W) from the centrifugal separation tank 30. The discharge mechanism 60 discharges the culture solution W from the centrifugal separation tank 30 through the suction pipe 62.

The suction pipe 62 has a first end portion 64. The first end portion 64 (its opening) is disposed closer to the center axis L than the culture solution W during centrifugation process and opposes the inner wall surface 32. In other words, the suction pipe 62 is disposed such that the distance between the first end portion 64 and the inner wall surface 32 is larger than the thickness of the culture solution W pressed against the inner wall surface 32 in a cylindrical shape. As a result, the first end portion 64 can be prevented from coming into contact with the culture solution W, the culture solution W can be maintained in the state of being centrifuged in the centrifugal separation tank 30, and the centrifugation supernatant of the culture solution W can be brought close to the first end portion 64.

The first end portion 64 can be configured to have a convex curved surface in the top view. Thus, the distance between the first end portion 64 and a liquid surface of the culture solution W can be adjusted, and the culture solution W (centrifugation supernatant) can be suctioned efficiently. In particular, the first end portion 64 can be configured to have a shape along the liquid surface of the culture solution W in the top view (not shown).

In the perfusion culture apparatus 1, the first end portion 64 is disposed in the vicinity of an upper end of the centrifugal separation tank 30 (adjacent to the collar portion 34) . However, this invention is not limited thereto, and the first end portion 64 can be disposed at any position from the vicinity of the bottom surface to the vicinity of the upper end of the centrifugal separation tank 30 in accordance with the processing object, procedure, etc.

The suction pipe 62 has a second end portion 66. The second end portion 66 is disposed outside the centrifugal separation tank 30, and the air and the culture solution W suctioned from the first end portion 64 are discharged from the second end portion 66. In this embodiment, the second end portion 66 is disposed within the pot 68. The pot 68 can be airtightly connected to the housing 40 via the suction pipe 62.

The discharge mechanism 60 includes a pump 70. The pump 70 serves to deliver the culture solution W (centrifugation supernatant) from the first end portion 64 to the second end portion 66. In this embodiment, the pump 70 is configured to apply a negative pressure in the pot 68 (to suction the air in the pot 68).

As a result, the air in the centrifugal separation tank 30 is suctioned from the first end portion 64, and the centrifugation supernatant of the culture solution W is discharged together with the air from the second end portion 66. The type of pump applicable to this embodiment is not particularly limited, but any known vacuum pump can be used, for example.

In this embodiment, the suction amount (suction amount per unit time) of the discharge mechanism 60 is set so that the centrifugation supernatant is suctioned from the liquid surface of the culture solution W while the suction pipe 62 is in close proximity to (but not in contact with) the liquid surface of the culture solution W. Thus, the centrifugation supernatant of the culture solution W can be discharged without interrupting the centrifugal state of the culture solution W. "Suction amount" refers to the amount of fluid (volume) suctioned per unit time. Namely, the fluid (air) of the volume set herein is suctioned by the pump 70 per unit time and discharged from the centrifugal separation tank 30.

In this embodiment, the perfusion culture apparatus 1 can be configured to include a recovery mechanism for recovering the culture solution W (centrifugal residue of culture solution W) from the centrifugal separation tank 30. In this embodiment, the recovery mechanism is realized as a return mechanism for recovering and returning the residue to the culture tank 10. Concretely, the perfusion culture apparatus 1 includes a return pipe 72 having one end disposed near the bottom surface of the centrifugal separation tank 30 and the other end disposed in the culture tank 10. Thus, for example, by increasing an internal pressure of the housing 40, the culture solution W (centrifugal residue of culture solution W) can be returned from the centrifugal separation tank 30 to the culture tank 10 through the return pipe 72.

Although the method of increasing an internal pressure of the housing 40 is not particularly limited, an internal pressure of the housing 40 can be increased by introducing sterile air into the pot 68 by using the pump 70. The other end of the return pipe 72 may be connected to another recovery container rather than the culture tank 10.

The perfusion culture apparatus 1 may be configured to include various other devices. For example, the perfusion culture apparatus 1 may be configured to include a control device for integrally controlling its operation, a communication device for communicating with an external device, a recording device, etc.

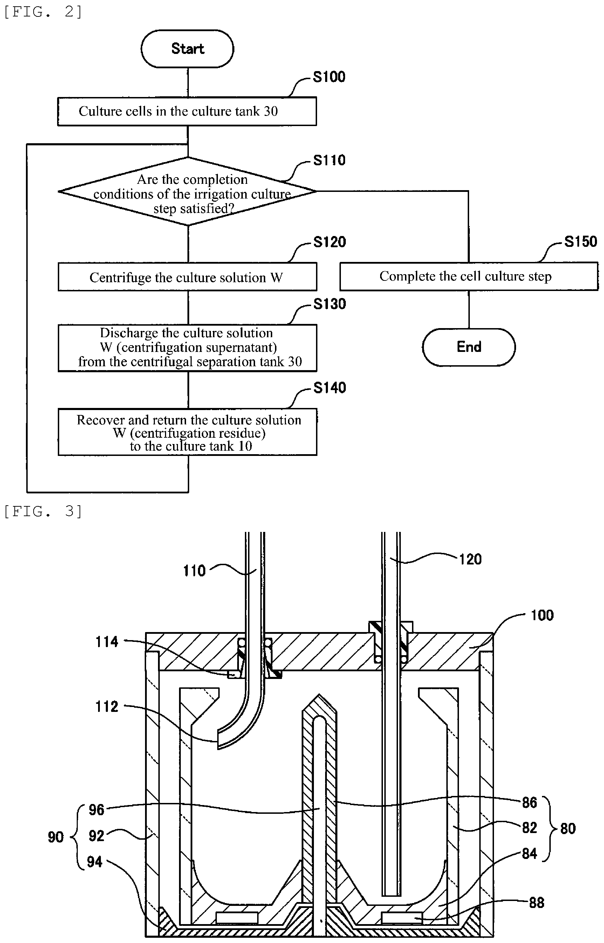

Next, an irrigation culture process according to an embodiment to which this invention is applied will be described with reference to FIG. 2.

The irrigation culture step according to this embodiment includes a step of culturing the cells dispersed in the culture solution W in the culture tank 10 (Step S100). This step can be realized by supplying the culture solution W to the culture tank 10 and seeding the cells, and maintaining the culture solution W in a desired state. Concretely, this step can be realized by controlling various kinds of ancillary equipment such that the temperature, dissolved oxygen concentration, dissolved carbon dioxide concentration, pH, cell density, etc. of the culture solution W in the culture tank 10 are at desired values. This step can also be performed while supplying the culture solution W (fresh liquid medium). Thus, even when the culture solution W is discharged from the culture tank 10 in a separation step of the culture solution W described below, an appropriate amount of the culture solution W can be maintained. This step is continued until completion conditions of the irrigation culture step are satisfied.

The irrigation culture step according to this embodiment includes a step of determining whether the completion conditions of the irrigation culture step are satisfied (Step S110).

Namely, in the irrigation culture step according to this embodiment, the step of culturing the cells dispersed in the culture solution W in the culture tank 10 is continued until the completion conditions of the irrigation culture process are satisfied (until Yes is achieved in Step S110), and thereafter, the steps of centrifuging the culture solution W (Step S120), discharging the culture solution W (Step S130), and returning the culture solution W (Step S140) are performed.

Various kinds of information can be used to determine the completion conditions of the culture step. For example, an index suitable for the target culture can be selected, such as the elapsed time from the start of the culture, the number of centrifugation steps and return steps described below, and the state of the culture solution W (amount and density of products and cells in culture solution W).

The culture solution W delivered from the culture tank 10 is centrifuged by rotating at a predetermined speed. Namely, the culture solution W delivered from the culture tank 10 and held in the centrifugal separation tank 30 is subjected to centrifugal force in the centrifugal separation tank 30 and pressed against the inner wall surface 32 to form a cylindrical shape. The culture solution W pressed against the inner wall surface 32 in the centrifugal separation tank 30 is affected by the centrifugal force, and components having a small specific gravity move closer to the rotation center, and components having a large specific gravity move closer to the inner wall surface 32. In the field of cell culture, cells are generally classified into components having a large specific gravity, so that by centrifugation of the culture solution W, the cells are concentrated closer to the inner wall surface 32 and the other components (metabolites of cells, etc., referred to as "centrifugation supernatant" as necessary) are concentrated closer to the rotation center.

The centrifugation process of the culture solution W may be performed while supplying the culture solution W from the culture tank 10 to the centrifugal separation tank 30, or after stopping the supply of the culture solution W.

The rotational speed of the centrifugal separation tank 30 in this step is not particularly limited, and can be set according to the processing object. The rotational speed of the centrifugal separation tank 30 may be kept constant, or may be changed during processing.

The irrigation culture step according to this embodiment includes a step of discharging the culture solution W (centrifugation supernatant of culture solution W) from the centrifugal separation tank 30 (Step S130). This step can be realized by suctioning the air in the centrifugal separation tank 30 from the first end portion 64 of the suction pipe 62 while rotating the centrifugal separation tank 30 at a predetermined speed. Namely, according to the perfusion culture apparatus 1, the first end portion 64 of the suction pipe 62 is disposed closer to the center axis L than the culture solution W in the centrifuged state, and opposes the inner wall surface 32 (culture solution W). In this state, by suctioning a necessary and sufficient amount of air from the first end portion 64, the culture solution W in the vicinity of the first end portion 64 can be suctioned together with the air and discharged from the second end portion 66.

As described above, the culture solution W is centrifuged in the centrifugal separation tank 30, and the centrifugation supernatant is concentrated to the side of the rotation center (vicinity of the first end portion 64), so that the centrifugation supernatant of the culture solution W is suctioned from the first end portion 64 of the suction pipe 62, and components having a large specific gravity such as cells remain in the centrifugal separation tank 30. It is also possible to aspirate a supernatant containing cells and adjust the cell density if necessary.

The step of discharging the culture solution W from the centrifugal separation tank 30 may be performed while supplying the culture solution W to the centrifugal separation tank 30, or may be performed after stopping the supply of the culture solution W. When the step is performed while supplying the culture solution W to the centrifugal separation tank 30, it is preferable that the suction amount of the discharge mechanism 60 is sufficiently larger than the supply speed of the culture solution W so that the culture solution W does not overflow the centrifugal separation tank 30.

In the step of discharging the culture solution W from the centrifugal separation tank 30, the rotational speed of the centrifugal separation tank 30 and the suction speed of the pump 70 may be kept constant, or may be changed on the way.

After confirming that the predetermined completion conditions are satisfied, the step of discharging the culture solution W is completed. The completion conditions are not particularly limited, and the discharge step can be completed according to, for example, processing time or discharge amount. The irrigation culture step according to this embodiment includes a step of recovering the residue remaining in the centrifugal separation tank 30 and returning the residue to the culture tank 10 (Step S140). This step can be realized by stopping the rotation of the centrifugal separation tank 30, dropping the residue to the bottom of the centrifugal separation tank 30, and thereafter supplying sterile air to the pot 68 by the pump 70. Namely, by supplying sterile air to the pot 68 to increase an internal pressure of the housing 40, the residue remaining in the centrifugal separation tank 30 can be delivered to the culture tank 10 through the return pipe 72.

While continuing the step of culturing cells in the culture tank 10 (Step S100), the step of centrifuging the culture solution W (Step S120), the step of discharging the culture solution W (Step S130), and the step of returning the culture solution W (Step S140) are repeated, and when the completion conditions of the irrigation culture step are satisfied (Yes in Step S110), the cell culture step is completed (Step S150), and the irrigation culture step is completed.

The step of returning the culture solution W (Step S140) may be performed each time the step of centrifuging the culture solution W (Step S120) and the step of discharging the culture solution W (Step S130) are performed, or may be performed after the step of centrifuging the culture solution W (Step S120) and the step of discharging the culture solution W (Step S130) are performed several times.

According to this embodiment, the culture solution W centrifuged in the centrifugal separation tank 30 is suctioned together with the air from the rotation center side of the centrifugal separation tank 30 and discharged. This makes it possible to discharge the centrifugation supernatant of the culture solution W without bringing the suction pipe 62 (first end portion 64) into contact with the liquid surface of the culture solution W in the centrifuging state (namely, the suction pipe 62 does not interrupt the centrifugation state of the culture solution W). Namely, according to this embodiment, it is possible to provide a perfusion culture apparatus which can easily discharge the centrifugation supernatant, has a simple configuration, and which is easy to handle.

Next, a modification of this embodiment will be described with reference to FIG. 3.

In this embodiment, the perfusion culture apparatus includes a centrifugal separation tank 80. The centrifugal separation tank 80 has a cylindrical side face portion 82 and a bottom surface portion 84. The centrifugal separation tank 80 also includes a central shaft 86. A magnet 88 is attached to the bottom surface portion 84, so that the centrifugal separation tank 80 can be rotated about the central shaft 86 (center axis of side surface portion 82).

The perfusion culture apparatus includes a housing 90. The housing 90 is a member for housing the centrifugal separation tank 80. The housing 90 has a cylindrical side surface portion 92 and a bottom surface portion 94. The housing 90 also includes a fixed shaft 96. By inserting the central shaft 86 of the centrifugal separation tank 80 into the fixed shaft 96, the centrifugal separation tank 80 can be rotatably held in the housing 90.

The housing 90 includes an upper lid 100. The upper lid 100 makes it possible to configure the housing 90 airtight. The upper lid 100 is provided with a plurality of through holes so that various members can be attached thereto.

A suction pipe 110 is attached to the housing 90. The suction pipe 110 is provided so that its end portion 112 is open toward the side surface portion 82 of the centrifugal separation tank 80 and opposes the side surface portion 82 at a predetermined distance.

In this embodiment, the suction pipe 110 is configured so that the distance between the end portion 112 and the side surface portion 82 can be adjusted. Concretely, the suction pipe 110 is attached to the upper lid 100 through an attachment 114 provided with a tapered through hole. Thus, an attachment angle of the suction pipe 110 can be adjusted in the attachment 114, and the distance between the end portion 112 and the side surface portion 82 can be changed.

The perfusion culture apparatus may be configured to be provided with an adjustment mechanism for adjusting the distance between the end portion 112 of the suction pipe 110 and the side surface portion 82 (not shown). The adjustment mechanism can be realized by a drive mechanism that moves the suction pipe 110 from outside the housing 90 to adjust the distance between the end portion 112 and the side surface portion 82. The adjustment mechanism may be configured such that the end portion 112 is brought close to the side surface portion 82 while suctioning the culture solution W and the air with the suction pipe 110. At this stage, the adjustment mechanism is preferably configured to adjust the behavior of the suction pipe 110 so that the end portion 112 (suction pipe 110) does not contact the liquid surface of the culture solution W.

In addition to the suction pipe 110, the housing 90 may be configured to have a return pipe 120 and an intake nozzle (not shown) for introducing sterile air.

With this perfusion culture apparatus, it is possible to adjust the distance between the end portion 112 of the suction pipe 110 and the side surface portion 82 (inner wall surface of the centrifugal separation tank 80). Therefore, even when the liquid surface position of the culture solution W changes by discharging the culture solution W from the centrifugal separation tank 80, the end portion 112 can be brought close to the liquid surface of the culture solution W in the centrifuged state, so that the culture solution W can be efficiently discharged.

In particular, by bringing the end portion 112 close to the side surface portion 82 while discharging the culture solution W, the end portion 112 can be kept close to the liquid surface of the culture solution W without bringing the end portion 112 into contact with the liquid surface of the culture solution W. Therefore, the culture solution W can be efficiently discharged without increasing the suction amount of the suction pipe 110.

Further, a predetermined amount of the culture solution W is supplied to the centrifugal separation tank 80 to carry out centrifugation for a predetermined amount of time, and thereafter the end portion 112 is brought close to the liquid surface of the culture solution W to discharge the culture solution W, so that the amount of time for carrying out the centrifugation can be sufficiently secured, and as a result, a good centrifugation can be carried out even when the culture solution W is difficult to centrifuge.

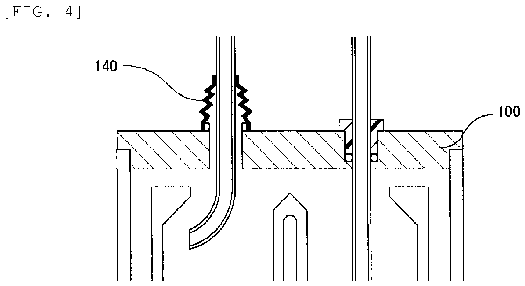

The mechanism for adjusting the distance between the end portion 112 of the suction pipe 110 and the side surface portion 82 is not limited to the above, and may be realized by other configurations. For example, as shown in FIG. 4, a bellows pipe 140 can be used to adjust the distance between the end portion 112 and the side surface portion 82. Namely, as shown in FIG. 4, the suction pipe 110 can be configured to pass through a through hole formed in the upper lid 100 and having an outer profile larger than that of the suction pipe 110, and the through hole can be covered with the bellows pipe 140. One end of the bellows pipe 140 is attached to the suction pipe 110, and the other end is attached to the upper lid 100 so as to cover the through hole. According to the above, the suction pipe 110 can change its posture inside the through hole, so that the distance between the end portion 112 and the side surface portion 82 can be adjusted, and the bellows pipe 140 can prevent contamination of the interior of the housing 90.

As another example, by configuring the suction pipe 110 to include a straight portion extending linearly and a bent portion bending and extending from a tip of the straight portion, and rotating the suction pipe 110 about the straight portion, it is possible to change the distance between the end portion 112 (tip of the bent portion) of the suction pipe 110 and the side surface portion 82 (not shown).

### [Industrial Applicability]

In the field of drug discovery and food products, cell culture techniques are used to produce a target product, and if the cell density in the culture solution can be increased, production efficiency of the target product can be enhanced.

As a technique for achieving a high-density culture of cells, a perfusion culture method is known in which a culture solution is taken out of a culture tank, metabolites are separated using a filtration membrane or centrifugal force, the removed cells are returned to the culture tank, and a fresh culture solution is added. However, in the case of utilizing a filtration membrane for cell separation, there is a possibility that the filtration membrane may be clogged, which is necessary to devise measures for its prevention for using the filtration membrane for long-term culture. Further, it was difficult to simplify the apparatus when centrifugal force is used to separate cells.

The culture apparatus of this invention provides a perfusion culture apparatus which can be used for long-term culture and has a simple mechanism and which is easy to handle, and a centrifugal separator.

### [Description of Reference Numerals]

- 1.: perfusion culture apparatus
- 10.: culture tank
- 20.: liquid delivery unit
- 22.: liquid delivery pipe
- 24.: first end portion
- 26.: second end portion
- 28.: pump
- 30.: centrifugal separation tank
- 32.: inner wall surface
- 34.: collar portion
- 36.: magnet
- 40.: housing
- 42.: shaft body
- 44.: filter
- 50.: drive mechanism
- 52.: magnet
- 54.: holding body
- 56.: motor
- 60.: discharge mechanism
- 62.: suction pipe
- 64.: first end portion
- 66.: second end portion
- 68.: pot
- 70.: pump
- 72.: return pipe
- 80.: centrifugal separation tank
- 82.: side surface portion
- 84.: bottom surface portion
- 86.: central shaft
- 88.: magnet
- 90.: housing
- 92.: side surface portion
- 94.: bottom surface portion
- 96.: fixed shaft
- 100.: upper lid
- 110.: suction pipe
- 112.: end portion
- 120.: return pipe
- 140.: bellows pipe
- W.: culture solution

## Claims

1. A perfusion culture apparatus comprising:
a centrifugal separation tank for centrifuging a culture solution delivered from a culture tank, and
a discharge mechanism for discharging a centrifugation supernatant of the culture solution from the centrifugal separation tank,
wherein the centrifugal separation tank has a cylindrical inner wall surface and is configured to centrifuge the culture solution by rotating about a center axis of the inner wall surface,
wherein the discharge mechanism includes a suction tube having a first end portion arranged closer to the center axis than the culture solution during a centrifugation process and opposing the inner wall surface, and a second end portion arranged outside the centrifugal separation tank, and
wherein, with the centrifugal separation tank being rotated and the culture solution being centrifuged, air in the centrifugal separation tank is suctioned from the first end portion of the suction tube, so that centrifugation supernatant of the culture solution is discharged from the second end portion together with the air.

2. The perfusion culture apparatus according to claim 1, wherein a centrifuging process of the culture solution is carried out while delivering the culture solution to the centrifugal separation tank.

3. The perfusion culture apparatus according to claim 1, wherein a centrifuging process of the culture solution is carried out while stopping delivery of the culture solution to the centrifugal separation tank.

4. The perfusion culture apparatus according to any of claims 1 to 3, further including a housing for storing the centrifugal separation tank configured to allow introduction of sterile air.

5. The perfusion culture apparatus according to any of claims 1 to 4, wherein the suction tube is configured to allow adjustment of a distance between the first end portion and the inner wall surface.

6. The perfusion culture apparatus according to claim 5, wherein the suction tube is configured to allow the first end portion to move toward the inner wall surface while suctioning the air.

7. The perfusion culture apparatus according to any of claims 1 to 6, further including a recovery mechanism for recovering a residue of the culture solution from the rotation tank.

8. A centrifugal separator comprising:
a centrifugal separation tank for centrifuging a processing solution, and
a discharge mechanism for discharging a centrifugation supernatant of the processing solution from the centrifugal separation tank,
wherein the centrifugal separation tank has a cylindrical inner wall surface and is configured to centrifuge the processing solution by rotating about a center axis of the inner wall surface,
wherein the discharge mechanism includes a suction tube having a first end portion arranged closer to the center axis than the processing solution during a centrifugation process and opposing the inner wall surface, and a second end portion arranged outside the centrifugal separation tank, and
wherein, with the centrifugal separation tank being rotated and the processing solution being centrifuged, air in the centrifugal separation tank is suctioned from the first end portion of the suction tube, so that centrifugation supernatant of the processing solution is discharged from the second end portion together with the air.
